(19) Europäisches Patentamt
European Patent Office
Office européen des brevets

(11) **EP 4 063 042 A1**

(12) **EUROPÄISCHE PATENTANMELDUNG**

(43) Veröffentlichungstag:
**28.09.2022 Patentblatt 2022/39**

(21) Anmeldenummer: **21164744.1**

(22) Anmeldetag: **24.03.2021**

(51) Internationale Patentklassifikation (IPC):
**B22F 3/06** (2006.01)  **A61C 7/12** (2006.01)
**A61F 2/28** (2006.01)  **A61F 2/30** (2006.01)
**A61K 6/844** (2020.01)  **B22F 3/12** (2006.01)
**B22F 3/26** (2006.01)  **B22F 10/28** (2021.01)
**C22C 1/04** (2006.01)  **C22C 1/05** (2006.01)
**C22C 29/12** (2006.01)

(52) Gemeinsame Patentklassifikation (CPC):
(C-Sets verfügbar)
**B22F 3/26; A61K 6/844; B22F 10/28; B33Y 10/00;
B33Y 70/00; B33Y 80/00; C04B 41/009;
C04B 41/5116; C04B 41/88; C22C 1/045;
C22C 1/05; C22C 29/12; C22C 33/0242;
C25D 1/00;** A61C 8/0013; (Forts.)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **MeKo
Laserstrahl-Materialbearbeitungen e.K.
31157 Sarstedt (DE)**

(72) Erfinder: **MEYER-KOBBE, Clemens
31157 Sarstedt (DE)**

(74) Vertreter: **Arth, Hans-Lothar
ABK Patent Attorneys
Jasminweg 9
14052 Berlin (DE)**

(54) **VERBUNDMATERIAL MIT SILBERINTEGRATION ALS ANTIBAKTERIELLER MATRIXWERKSTOFF INSBESONDERE ZUR BIOFILMREDUKTION**

(57) Die vorliegende Erfindung betrifft ein Verbundmaterial aus einem Matrixwerkstoff und Silber, wobei das Silber homogen in dem Matrixwerkstoff verteilt ist und der Gehalt an Silber im Verbundmaterial zwischen 5 Gew.-% und 40 Gew.-% liegt sowie Verfahren zur Herstellung dieses Verbundmaterials und medizinische Implantate enthaltend dieses Verbundmaterial. Das Verbundmaterial ist insbesondere zur Reduktion und Vermeidung von Biofilmbildung geeignet.

Figur 3

**EP 4 063 042 A1**

(52) Gemeinsame Patentklassifikation (CPC): (Forts.)
A61C 2008/0046; A61F 2/28; A61F 2/30;
A61F 2/38; A61F 2/44; A61F 2002/30736;
C04B 2111/00836; C22C 33/0285

C-Sets
**C04B 41/009, C04B 35/10, C04B 38/00;**
**C04B 41/009, C04B 35/48, C04B 38/00;**
**C04B 41/5116, C04B 41/4523**

## Beschreibung

Die vorliegende Erfindung betrifft ein Verbundmaterial aus einem Matrixwerkstoff und Silber, wobei das Silber homogen in dem Matrixwerkstoff verteilt ist und der Gehalt an Silber im Verbundmaterial zwischen 5 Gew.-% und 40 Gew.-% liegt sowie Verfahren zur Herstellung dieses Verbundmaterials und medizinische Implantate enthaltend dieses Verbundmaterial. Das Verbundmaterial ist insbesondere zur Reduktion und Vermeidung von Biofilmbildung geeignet.

Die vorliegende Erfindung beschreibt ferner ein antibakterielles Verbundmaterial für medizinische Implantate, vorzugsweise medizinische Langzeitimplantate insbesondere zur Anwendung als Bracketmaterial in der Kieferorthopädie. Es wird keine oberflächliche Beschichtung aufgebracht, sondern antibakterielles Silber mittels einer Integration z.B. in die Poren eines Sinterlings aus Metall oder Keramik eingebracht. Das Silber bildet dabei keine geschlossene Oberfläche, besitzt aber eine ausreichende Fernwirkung, um eine Biofilmanhaftung großflächig zu verhindern.

## Stand der Technik

Die Biofilmbildung im oralen Milieu ist eines der zentralen Probleme in der Zahnmedizin. Mikroorganismen lösen Karies, Parodontopathien und andere Zahnerkrankungen aus, welche die hauptsächlich zu behandelnden Erkrankungen in der Zahnmedizin darstellen.

Unzureichende Mund- und Zahnhygiene und die damit einhergehende Biofilmbildung führt bei Patienten aller Altersgruppen zu Zahnkaries, auch kurz Karies genannt. Gemäß Wikipedia ist Karies "eine multifaktoriell bedingte destruierende Erkrankung der Zahnhartgewebe, Zahnschmelz und Dentin. Sie entsteht unter Beteiligung von Mikroorganismen und geht von einer durch Säureeinwirkung "entkalkten" Zahnoberfläche aus."

Die Biofilmbildung begünstigt außerdem die Entstehung von Parodontitis, also einer entzündlichen Erkrankung des gesamten Zahnhalteapparates inklusive dem Kieferknochen. Insbesondere erwachsene Patienten sind durch das Anhaften von Plaque an intraoralen Oberflächen einem erhöhten Parodontitis-Risiko ausgesetzt. In der Folge können chronische Parodontitiden entstehen oder sich verstärken, die bei Nichtbehandlung zu Zahnlockerungen durch Attachmentverlust und in Extremfällen letztlich zu Zahnverlust führen können.

Wissenschaftlich ist auch eine enge Assoziation dieser oralen Plaqueakkumulation mit systemischen Erkrankungen des Körpers nachgewiesen, wie beispielsweise arteriosklerotische Veränderungen der blutführenden Gefäße.

Die unerwünschte Biofilmbildung ist auch in der Kieferorthopädie von immenser Bedeutung und betrifft insbesondere die große Patientengruppe von Jugendlichen im pubertären Alter, deren Motivation im Bereich der Zahnpflege häufig verhalten ausfällt. Erschwerend kommt hinzu, dass durch pubertäre Hormonumstellungen junge Patienten anfälliger gegenüber Gingivitis sind, einer bakteriell verursachten Entzündung des marginalen Zahnfleisches.

Zunehmend wünschen auch erwachsene Patienten eine Korrektur ihrer Zahnfehlstellung, da der Stellenwert einer kieferorthopädischen Behandlung von Zahn- und Kieferfehlstellungen in der Gesellschaft deutlich zugenommen hat. Dieser Zuwachs ist zum einen mit dem steigenden Gesundheitsbewusstsein der Bevölkerung und zum anderen mit den erweiterten therapeutischen Möglichkeiten im Bereich der Kieferorthopädie zu erklären. Behandlungen mit vollständig individuellen lingualen Apparaturen ermöglichen mittlerweile eine präzise kieferorthopädische Zahnstellungskorrektur nahezu ohne ästhetische Beeinträchtigungen.

Festsitzende kieferorthopädische Apparaturen, unabhängig von einer bukkalen oder lingualen Insertion, erfordern aufgrund der komplexen Geometrie einen hohen Reinigungsaufwand. Damit einhergehend ist eine ausgesprochen gute Compliance des Patienten nötig, wenn das Auftreten unerwünschter Nebenwirkungen wie die Demineralisation des Zahnschmelzes, Entstehung von Karies oder parodontale Veränderungen reduziert werden soll. Insbesondere wird die Entstehung von Demineralisationen (Dekalzifikationen) des Zahnschmelzes als Folge der Biofilmbildung im oralen Milieu als zentrales Problem der Kieferorthopädie angesehen.

Da die Fallzahlen von behandelten Patienten mit festsitzenden Apparaturen und das Patientenalter ansteigen, liegt ein wesentlicher Fokus der klinischen Forschungstätigkeit darin, das Auftreten solcher, durch ärztliche Behandlung entstandener (iatrogene) Effekte während der kieferorthopädischen Therapie zu vermeiden.

Bis dato ist selbst bei sehr guter Patientencompliance die Biofilmvermeidung auf kieferorthopädischen Behandlungsapparaturen nicht möglich. Die Zahnreinigung ist bei kieferorthopädischen Apparaturen, insbesondere bei festsitzenden Behandlungsapparaturen, erschwert. Festsitzende kieferorthopädische Behandlungsapparaturen, wie z.B. Brackets, weisen aufgrund ihrer Geometrie schwer zu reinigende Nischen auf, was bei insuffizienter Mundhygiene zu Demineralisationen des Zahnschmelzes im Bracketumfeld führen kann.

Mit der mehrmals täglichen, gründlichen Reinigung der Zähne kann man der Biofilmbildung entgegenwirken, ohne diese jedoch ganz beseitigen zu können. Ein dünnes Zahnschmelzoberhäutchen aus adsorbierten Proteinen ermöglicht bakteriellen Frühbesiedlern unmittelbar nach einer mechanischen Entfernung des Biofilms die Anhaftung auf dem Zahnschmelz. Im Folgenden wächst die Plaque durch Akkumulation von Spätbesiedlern weiter an. Die so entstehende dreidimensionale Biofilmstruktur aus Bakterien, eingebettet in einer Matrix aus extrazellulären Polysacchariden, kann im Verhältnis zueinander sehr unterschiedlich sein. Je nach Bakterienart und den umgebenden Bedingungen differiert der Bakterienanteil in der Matrix entweder zwischen 2 - 15 Vol.% oder 60 - 70 Vol.%. In diesem Zustand ist der Biofilm durch die Selbstreinigungseffekte der Mundhöhle nicht mehr zu beseitigen. Die Bakterien verstoffwechseln zugeführte Kohlenhydrate zu organischen Säuren und ein saures anaerobes Milieu entsteht. In der Folge werden Calcium- und Phosphationen aus der Kristallgitterstruktur des Zahnschmelzes herausgelöst. Langfristig werden diese Dekalzifikationen des Zahnschmelzes als irreversible weiße Flecken, sogenannte "white spot lesions" sichtbar.

Die "White spot lesions" sind das initiale Stadium einer kariösen Läsion des Zahnes. Bei anhaltendem sauren Milieu und fortschreitenden Demineralisationsvorgängen mündet die initiale Schmelzkaries in eine Dentinkaries.

Erschwerend kommt hinzu, dass eine reguläre festsitzende kieferorthopädische Therapie in der Regel ca. 18 - 24 Monate dauert. Über diesen gesamten Behandlungszeitraum sind die Brackets auf den Zähnen fixiert und mittels Bögen und Ligaturen miteinander verbunden. Das kieferorthopädische Material, welches zu Behandlungsbeginn inseriert wurde, ist also über einen sehr langen Zeitraum in der Mundhöhle exponiert und den intraoralen Bedingungen ausgesetzt.

In der aktuellen medizinischen und zahnmedizinischen Forschung besteht das Bestreben, diese mikrobielle Besiedlung auf Werkstoffoberflächen in der Mundhöhle ab initio, d.h. ab Insertionszeitpunkt, zu vermeiden.

Um die unerwünschten klinischen Nebenwirkungen von festsitzenden Behandlungsapparaturen zu minimieren, gibt es verschiedene Strategien. Im Zuge des steigenden Gesundheitsbewusstseins gewinnen dabei präventive Behandlungskonzepte zur Biofilmreduzierung zunehmend an Bedeutung.

Generell wird angestrebt, dem günstigen Vermehrungsmilieu für Mikroorganismen im Mund durch verbesserte orale Hygiene zu begegnen oder dentale Werkstoffe mit verminderter Biofilmaffinität (jeweils abhängig vom Material und deren Oberfläche) auszuwählen oder die kieferorthopädischen Apparaturen mit bakteriziden Eigenschaften zu versehen.

Die verschiedenen existierenden Konzepte, um Demineralisationen des Zahnschmelzes während einer kieferorthopädischen Behandlung zu reduzieren, werden im Folgenden dargestellt.

a) Verbesserung der individuellen Mundhygiene

Ein präventiver Ansatz liegt in der Patientenmotivation und -lenkung. Hier steht die Verbesserung der häuslichen Mundhygiene im Vordergrund. Darüber hinaus ist die Reduktion des oralen Biofilms im Rahmen der halbjährlich durchzuführenden professionellen Zahnreinigung von großer Bedeutung. Außerdem kann eine Erhöhung der Säureresistenz des Zahnschmelzes durch regelmäßige häusliche und in-office Fluoridierung erreicht werden. Es wird die Anwendung antimikrobieller bzw. fluoridhaltiger Mundspüllösungen empfohlen, mittels derer die Biofilmbildung merklich reduziert werden kann. Ziel ist unter anderem die effektive Reduzierung des kariogenen Bakteriums Streptococcus mutans sowie die hemmende Wirkung gegenüber Aggregatibacter actinomycetemcomitans.

Wie zuvor erläutert, lässt sich jedoch selbst bei guter Patientencompliance und einer guten Mundhygiene eine Biofilmbildung nur verringern, aber nicht vermeiden.

b) Fluoridhaltige orthodontische Klebstoffe

Zum Einsatz kommen Befestigungsadhäsive (Klebstoffe zur Befestigung der Brackets), die mit Fluorid-Nanopartikeln versetzt sind. Diese fluoridhaltigen Befestigungsmaterialien führen zu einer erhöhten Widerstandsfähigkeit der Zahnhartsubstanz gegen ein saures anaerobes Milieu und somit zur geringeren Demineralisation des Schmelzes. So konnte im Bereich der fluoridhaltigen Befestigungskomposite eine Tendenz zu einer geringeren Demineralisation des Schmelzes festgestellt werden.

Nanopartikel sind aufgrund ihrer Größe zellgängig und werden mangels der bereits bekannten Nebenwirkungen und der vielfach unzureichend erforschten Risiken jedoch kritisch betrachtet.

c) Modifizierung des direkten Bracketumfelds

Mittels einer Versiegelung des Zahnschmelzes im unmittelbaren Bracketumfeld kann eine mechanisch-chemische Barriere gegen den Säureangriff geschaffen werden. Die Versiegelungen sind aber wenig abrasionsbeständig und weisen daher nur eine vorübergehende Wirkung auf.

d) Beschichtungen von Bracket-Apparaturen

Antibakterielle Beschichtungen von Brackets verhindern eine Biofilmbildung von Anbeginn bei gleichzeitig guter Biokompatibilität. Die Beschichtungen werden vollflächig aufgebracht, d.h. die antibakteriellen Deckschichten haben eine lückenlos geschlossene Oberfläche, womit das Grundmaterial, also das eigentliche Bracketmaterial eingekapselt ist.

Ein Verfahren ist das Versiegeln kieferorthopädischer Apparaturen durch Polytetrafluorethylen (PTFE), um das Anhaften von Polysacchariden zu unterbinden. Die Bildung eines adhärenten Biofilms kann dabei stark minimiert werden.

Durch die vorherrschenden oralen Bedingungen kommt es jedoch zur schnellen Abrasion oberflächlich aufgetragener Versiegelungen und somit zu einem raschen Verlust des antibakteriellen Effekts.

Alternativ eignen sich galvanisch oder mittels des Verfahrens der physical vapor deposition (PVD) aufgetragene antibakteriell wirkende Beschichtungen. Häufig wurde dabei Silber als biokompatibler und antibakterieller Werkstoff genutzt.

In einer Tierstudie an Zähnen von Ratten wurde nachgewiesen, dass silberbeschichtete Oberflächen von kieferorthopädischen Apparaturen ohne zusätzliche Mundhygiene durch gute Patientencompliance einen antibakteriellen Effekt haben. Die Studie zeigt, dass die Beschichtung zu einer verminderten Entstehung von Streptococcus mutans führt und somit das Kariesrisiko reduziert. Generell werden Beschichtungen im Mund aber abgerieben und haben nur eine vorübergehende Wirkung.

Außer reinem Silber wurden Untersuchungen zur Wirkung von Silbernanopartikeln in der Zahnmedizin und der Kieferorthopädie durchgeführt. Es konnte nachgewiesen werden, dass das Hinzufügen von 5 % oder 10 % Silber/Hydroxyapatit-Nanopartikeln in kieferorthopädische Befestigungsadhäsive ein weiteres Bakterienwachstum hemmt und somit in unmittelbarer Umgebung des Adhäsivs antibakteriell wirkt. Das Hinzufügen von Silbernanopartikeln in Adhäsiven führt dabei zu einer Inhibierung des weiteren Wachstums von Streptococcus mutans.

Silbernanopartikel werden jedoch nicht nur in Befestigungsadhäsive oder -zemente eingebracht, sondern auch als Beschichtung benutzt. Es wurde nachgewiesen, dass auf Silbernanopartikel-beschichtetem Dentin die Bakterienadhäsion verhindert werden kann.

Silbernanopartikel weisen eine Größe von bis zu 100 nm auf und besitzen damit die Fähigkeit auch außerhalb von festen und flüssigen Festkörperzuständen aufzutreten. Sie können zelltoxisch wirken, indem sie den Zellzyklus, die DNA und die Zellapoptose beeinflussen. Silbernanopartikel haben auf humane mesenchymale Stammzellen ab einer bestimmten Konzentration einen zell- und gentoxischen Charakter und verursachen eine DNA-Beschädigung, Zellsterben sowie funktionelle Beeinträchtigungen.

Trotz der zahlreichen positiven Studien, die eine antibakterielle Wirkung von Silbernanopartikeln belegen, ist daher der kommerzielle Einsatz im medizinischen Bereich aufgrund zahlreicher Untersuchungen, die eine Gesundheitsgefährdung durch Nanopartikel aufzeigen, kritisch zu sehen.

Wie zuvor beschrieben, weisen reine Silberbeschichtungen aber den Nachteil einer geringen Abrasionsbeständigkeit auf. Problematisch für oberflächlich aufgetragene Beschichtungen innerhalb der Mundhöhle ist die hohe intraorale Abrasionsbelastung durch starke Scher- und Muskelkräfte der Zähne, Zunge und perioralen Muskulatur sowie der Angriff durch verschiedene Flüssigkeiten in der Mundhöhle. Durch partielle Schädigungen oder flächigen Abtrag der in der Regel sehr dünnen Beschichtungen geht die antibakterielle Wirkung schnell verloren. Die antibakterielle Schutzwirkung ist somit nicht von Dauer.

Der gravierende Nachteil aller Oberflächenbeschichtungen in der Zahnheilkunde ist folglich die hohe Abrasion in der Mundhöhle, die bereits nach kurzer Zeit zum Abrieb der Beschichtungen führt.

Für die Behandlungsapparaturen in der Kieferorthopädie existieren derzeit keine Werkstoffe oder Beschichtungstechniken mit einer zufriedenstellenden, langanhaltenden antibakteriellen Wirkung, um die Entstehung des initialen intraoralen Biofilms zu verhindern. Alle aufgezeigten Präventionskonzepte können eine erhöhte Plaqueakkumulation über einen längeren Zeitraum nicht soweit hemmen, dass bei Multibracketapparaturen das zentrale Problem der Entstehung von "white spot lesions" unterbleibt.

Aus den genannten Gründen bedarf es der Entwicklung eines medizinischen Implantats, welches sowohl die Entstehung des Biofilms, z.B. auf kieferorthopädischen Apparaturen intraoral direkt verhindert, als auch über eine gute Widerstandsfähigkeit gegen Scherkräften und Abrasionen verfügt.

Aufgabe der vorliegenden Erfindung ist es somit, ein Material für ein Implantat und ein Implantat bereitzustellen, welches die Entstehung eines Biofilms verhindert oder zumindest deutlich entgegenwirkt bei gleichzeitiger Widerstandsfähigkeit gegen Scherkräfte und Abrasionen, um eine dauerhafte Schutzwirkung zu erzielen.

Diese Aufgabe wird erfindungsgemäß durch die technische Lehre der unabhängigen Ansprüche gelöst. Weitere vorteilhafte Ausgestaltungen der Erfindung ergeben sich aus den abhängigen Ansprüchen, der Beschreibung, den Figuren sowie den Beispielen.

Es wurde gefunden, dass Beschichtungen und selbst unüblich dicke Beschichtungen eines Implantats mit einem antibakteriellen Material oder Wirkstoff nur kurzzeitig vor der Entstehung eines Biofilms schützen, da derartige Beschichtungen unzureichend stabil gegenüber einwirkenden Kräften sind und abgerieben werden.

Überraschend wurde jedoch gefunden, dass eine vollständige Beschichtung eines Implantats mit einem antibakteriellen Material oder Wirkstoff gar nicht erforderlich ist, sondern eine homogene Verteilung von Silber in einem Matrixwerkstoff zu einem Verbundmaterial führt, das dauerhaft vor einer Biofilmbildung auf dem Verbundmaterial schützt und dieser Schutz auch nicht durch auf das Verbundmaterial einwirkende Kräfte geschmälert wird.

Somit betrifft die vorliegende Erfindung ein Verbundmaterial aus einem Matrixwerkstoff und Silber, wobei das Silber homogen in dem Matrixwerkstoff verteilt ist und der Gehalt an Silber im Verbundmaterial zwischen 5 Gew.-% und 40 Gew.-% liegt.

**Matrixwerkstoff**

Der Begriff >>Matrixwerkstoff<< wie hierin verwendet bezeichnet das Grundmaterial, in welches das Silber homogen verteilt eingelagert wird. Erfindungsgemäß finden in der Medizin und Medizintechnik eingesetzt Metalle, Metalllegierungen und Keramiken als Matrixwerkstoff Verwendung. Der Matrixwerkstoff selbst hat keine antibakteriellen Eigenschaften.

Als Matrixwerkstoff sind grundsätzlich alle Metalle, Metalllegierungen und Keramiken, mit folgenden Eigenschaften geeignet:

a) Biokompatibilität (korrosionsbeständig, hämokompatibel, nicht zyto- und genotoxisch, nicht karzinogen),
b) eine für Dentalimplantate übliche Abrasionsbeständigkeit,
c) Sinterfähigkeit zur Herstellung von porösen Werkstoffen,
d) Schmelztemperatur oberhalb der Schmelztemperatur von Silber (961,8°C),
e) geringe Sprödigkeit, d.h. eine ausreichende Duktilität, um bei schlagartiger Beanspruchung nicht zu brechen.

Als Beispiele für bevorzugte Matrixwerkstoffe kommen vorzugsweise für Implantate zugelassene Metalle und Metalllegierungen in Frage wie Edelstähle (z.B. 316LVM®, 316Lmedical), Co-Basis-Legierungen bzw. CoCr-Werkstoffe (z.B. L605®, Phynox®, MP35N®), Nickel-freie Eisen-Chrom-Mangan-Legierungen wie z.B. Vasculoy®, Cobalt-Nickel-Chrom-Legierungen wie z.B. Duratherm®, Eisen-Chrom-Nickel-Legierungen wie z.B. Durinox®, Eisen-Cobalt-Nickel-Legierungen wie z.B. Durnico® und Phytime®, Titanlegierungen, Tantal und Wolfram sowie Keramiken aus Aluminiumoxid oder Zirkoniumdioxid.

Zusammensetzung von Vasculoy® (Angaben in Gew.%)

| Cr | Mn | Mo | N | C | P | S | Fe |
|---|---|---|---|---|---|---|---|
| 14,0-16,5 | 10,0-12,0 | 3,0-4,0 | ≤0,70 | ≤0,20 | ≤0,02 | ≤0,02 | Rest |

Zusammensetzung von Duratherm® 600 (Angaben in Gew.%)

| Co | Ni | Cr | Fe | W | Mo | Ti | Al |
|---|---|---|---|---|---|---|---|
| 42 | 26 | 12 | 9 | 4 | 4 | 2 | 1 |

Zusammensetzung von Durinox® (Angaben in Gew.%)

| Cr | Ni | Mo | Ti | Mn | Si | C | P | S | Fe |
|---|---|---|---|---|---|---|---|---|---|
| 8,5-10,0 | 8,5-11,0 | 4,5-5,5 | 0,5-1,0 | ≤0,30 | ≤0,30 | ≤0,03 | ≤0,025 | ≤0,015 | Rest |

Zusammensetzung von Durnico® (Angaben in Gew.%)

| Co | Ni | Mo | Ti | Al | Mn | Si | C | P | S | Fe |
|---|---|---|---|---|---|---|---|---|---|---|---|
| 8,0-10,0 | 17,0-19,0 | 4,5-5,5 | 0,5-0,8 | 0,05-0,15 | ≤0,10 | ≤0,10 | ≤0,03 | ≤0,01 | ≤0,01 | Rest |

Zusammensetzung von Phytime® (Angaben in Gew.%)

| Ni | Co | Mo | Si | C | P | S | Fe |
|---|---|---|---|---|---|---|---|
| 18 | 16 | 5 | ≤0,30 | ≤0,03 | ≤0,02 | ≤0,02 | Rest |

Als Matrixwerkstoff sind somit Metalle und Metalllegierungen, vorzugsweise austenitische Metalle und austenitische Metalllegierungen geeignet. Beispiele sind Edelstähle, CoCr-Legierungen, Eisen-Chrom-Mangan-Legierungen, Cobalt-Nickel-Chrom-Legierungen, Eisen-Chrom-Nickel-Legierungen, Eisen-Cobalt-Nickel-Legierungen, Titanlegierungen, Tantal und Wolfram sowie Keramiken aus Aluminiumoxid und Keramiken aus Zirkoniumdioxid. Die Metalle, Metalllegierungen und Keramiken sind als Werkstoff für Dentalimplantate geeignet.

Der Begriff >>Abrasionsbeständigkeit<< wie hierin verwendet bezeichnet eine hohe Widerstandsfähigkeit gegen Abrasion, so dass Abschabungen und Abtragungen nur in geringem und im Dentalbereich üblichen Maße erfolgen. Ein gewisser Abrieb ist tolerabel und auch unvermeidbar. Ein erfindungsgemäßer Vorteil ist aber, dass das Silber im Matrixwerkstoff homogen verteilt ist, so dass auch bei Abrieb immer wieder Silber an die Oberfläche kommt, welches die Biofilmbildung verhindert.

Der Begriff >>Sinterfähigkeit<< wie hierin verwendet ist wie folgt definiert:

Die Fähigkeit aus feinkörnigen keramischen oder metallischen Stoffen unter hohen Temperaturen (jedoch unterhalb der Schmelztemperaturen der Hauptkomponenten) und oft unter Druck ein Werkstück zu formen, so dass die Gestalt des Werkstückes erhalten bleibt.

Der Begriff >>Sprödigkeit<< wie hierin verwendet ist wie folgt definiert:

Sprödigkeit ist eine Werkstoffeigenschaft, die das Versagens- bzw. Bruchverhalten beschreibt. Ein spröder Werkstoff lässt sich nur im geringen Maße plastisch verformen und zeichnet sich folglich durch geringe Duktilität aus.

**Silber als antibakterieller Füllstoff**

Es ist nachgewiesen, dass Silber in Kontakt mit Blut oder Gewebe eine antibakterielle und heilungsfördernde Wirkung aufweist. Der antibakterielle Effekt des Silbers beruht auf der Permeabilisierung der bakteriellen Zellmembran und der Bildung von intrazellulär schwer löslichen Komplexen mit bakteriellen Enzymen. Dies führt zur Zerstörung der bakteriellen Atmungskette und zur Hemmung der DNA-Replikation der Mikroorganismen.

Durch die Integration von Silber in den Matrixwerkstoff kann das Silber nicht vollständig durch Abrasion abgetragen werden wie es bei einer Beschichtung aus Silber oder enthaltend Silber der Fall wäre. Vielmehr befindet sich konstant eine ausreichende Silberkonzentration an der Oberfläche des Verbundmaterials, sodass langfristig eine Biofilmbildung verhindert oder zumindest aber stark reduziert wird.

Erfindungsgemäß muss das Silber dafür homogen in dem Matrixwerkstoff verteilt sein, so dass nicht nur die Oberfläche des Matrixwerkstoffs antibakteriell wirkt, sondern ein Verbundmaterial entsteht, welches insgesamt also nicht nur an seiner Oberfläche sondern durch das gesamte Material hindurch antibakteriell ist.

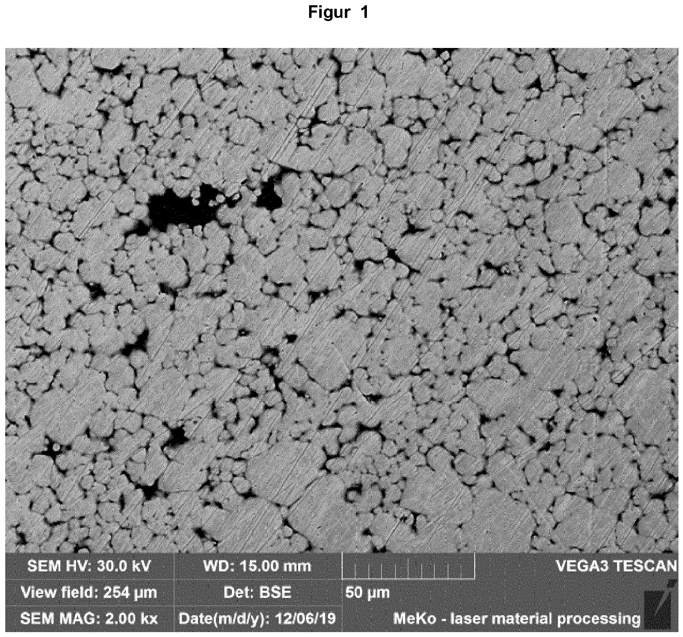

Dies veranschaulicht Figur 1, welche eine Rasterelektronenmikroskopaufnahme eines Querschliffes eines porösen Wolfram-Matrixwerkstoffs zeigt. Schwarz dargestellt wird das eingelagerte Silber. Die homogene Verteilung des Silbers im gesamten Wolfram-Matrixwerkstoff ist klar zu erkennen.

Der Begriff >>homogen<< wie hierin verwendet bedeutet, dass pro willkürlich ausgewählter Volumeneinheit von Verbundmaterial die gleiche Masse an Matrixwerkstoff und die gleiche Masse an Silber enthalten sind. Die Abweichungen bezüglich der Masse an Matrixwerkstoff und der Masse an Silber zwischen zwei willkürlich gewählten Volumeneinheiten des Verbundmaterials betragen nicht mehr als 20%. Veranschaulicht bedeutet dies folgendes. Wenn in einer willkürlich gewählten Volumeneinheit des Verbundmaterials von 0,1 mm$^3$ 90 Gew.-% Matrixwerkstoff und 10 Gew.-% Silber enthalten sind, in jeder anderen Volumeneinheit des Verbundmaterials von 0,1 mm$^3$ zwischen 8 Gew.-% und 12 Gew.-% Silber enthalten sein müssen.

Der Gehalt an Silber oder genauer der Gehalt an homogen verteiltem Silber im Verbundmaterial liegt zwischen 4 Gew.-% und 40 Gew.-%, bevorzugt zwischen 5 Gew.-% und 35 Gew.-%, weiter bevorzugt zwischen 6 Gew.-% und 30 Gew.-%, weiter bevorzugt zwischen 7 Gew.-% und 25 Gew.-%, weiter bevorzugt zwischen 8 Gew.-% und 20 Gew.-%, weiter bevorzugt zwischen 9 Gew.-% und 17 Gew.-% und am meisten bevorzugt zwischen 10 Gew.-% und 15 Gew.-% Silber bezogen auf das Verbundmaterial.

**Verbundmaterial**

Als >>Verbundmaterial<< wie hierin verwendet, wird die Kombination aus Matrixwerkstoff und Silber bezeichnet, wobei eine bestimmte Menge an Silber homogen in dem Matrixwerkstoff verteilt ist. Damit erhält man ein antibakterielles Verbundmaterial, welches nicht nur an seiner Oberfläche antibakteriell ist sondern auch an jeder beliebigen Stelle innerhalb des Verbundmaterial. Das Verbundmaterial im medizinischen Implantat weist somit keine antibakterielle Beschichtung auf.

Das Verbundmaterial ist somit ein neuer homogener Werkstoff aus Matrixwerkstoff und darin homogen verteiltem Silber und kein Matrixwerkstoff mit einer antibakteriellen Beschichtung. Damit bleibt auch bei Abrieb und selbst bei starkem Abrieb die antibakterielle Eigenschaft des Verbundmaterials erhalten, da bei Abrieb immer wieder Silber an die Oberfläche des Verbundmaterials kommt und dadurch die antibakterielle Wirkung dauerhaft aufrecht erhält.

Daher sind Beschichtungen als antibakterielle Oberflächen aus den vorgenannten Gründen ungeeignet, eine antibakterielle Langzeitwirkung zu garantieren. Das erfindungsgemäße Verbundmaterial vereint beide Eigenschaften in idealer Weise. Es besteht aus einem porösen Matrixwerkstoff mit integriertem Silber als Intramatrix-Füllstoff. Der Matrixwerkstoff sorgt für die Stabilität und verleiht dem Verbundmaterial einen hohen Abrasionswiderstand und das integrierte homogen verteilte Silber sorgt für die antibakterielle Wirkung nicht nur an der Oberfläche des Verbundmaterials und hält auch bei starker Abrasion die antibakterielle Wirkung aufrecht.

Das erfindungsgemäße Verbundmaterial dient somit zur Reduzierung oder Verhinderung der Ausbildung und Anhaftung eines Biofilms an der Oberfläche des Verbundmaterials. Vor allem dient das Verbundmaterial zur dauerhaften Reduzierung oder Verhinderung der Ausbildung und Anhaftung eines Biofilms an der Oberfläche des Verbundmaterials so lange das Verbundmaterial sich im Körper befindet.

**Herstellung des Verbundmaterials mit homogener Silberintegration**

Zur Herstellung des Verbundmaterials sind vor allem folgende Verfahren geeignet.

Sinterprozess:

Aus feinkörnigem Metall- oder Keramikpulver oder auch aus einer Mischung verschiedener Körnungen eines Metall- oder Keramikpulvers wird ein Sinterrohling gepresst. Der Sinterrohling hat zumeist bereits die Form des gewünschten Werkstücks oder Formteils. Durch Wärmebehandlung mit oder ohne Druckaufbringung unterhalb der Schmelztemperatur, dem eigentlichen Sinterprozess, werden die Pulverpartikel miteinander verbunden und der Sinterrohling zum "Sinterling" verdichtet und ausgehärtet. Die Ausgangsstoffe werden, umgangssprachlich formuliert, "zusammengebacken". Der gesinterte Matrixwerkstoff, d.h. der Sinterling muss eine offene Porosität aufweisen.

In einem Folgeprozess wird der Sinterling unter Unterdruckbedingungen in geschmolzenes Silber getaucht und so Silber in das offenporige Sintermaterial infiltriert. Durch den Unterdruck wird sichergestellt, dass das Silber in sämtliche Poren des Sinterlings eindringt und diese füllt.

Die Porosität des Sinterlings muss so groß sein, dass Silber den Matrixwerkstoff infiltrieren kann und anschließend genügend Silber im Matrixwerkstoff bzw. im Verbundmaterial vorhanden ist, um eine antibakterielle Wirkung sicherzustellen. Andererseits soll die Porosität auch nur so groß sein, dass möglichst wenig teures Silber verbraucht wird und die Abrasionsbeständigkeit des Matrixwerkstoffes erhalten bleibt oder nur unwesentlich reduziert wird.

Eine Porosität des Matrixwerkstoffes von weniger als 30%, vorzugsweise von unter 20%, weiter bevorzugt von unter 15% hat sich als vorteilhaft erwiesen. Bei einer Porosität von 5% - 10%, entsprechend bei Silbergehalten von 5% - 10% ist die antibakterielle Wirkung verringert und unterhalb von 5% nahezu nicht vorhanden.

Der Begriff >>Porosität<< wie hierin verwendet ist wie folgt definiert:

Die Porosität ist eine dimensionslose Größe und stellt das Verhältnis von Hohlraumvolumen $V_H$ zu Gesamtvolumen V eines Stoffes oder Stoffgemisches dar. Sie dient als klassifizierendes Maß für die tatsächlich vorliegenden Hohlräume. Die Porosität kann durch folgende Formel dargestellt werden

$$\Phi = \frac{V_H}{V} = \frac{V_H}{V_H + V_F}$$

worin $V_F$ das Reinvolumen des Feststoffes bezeichnet.

Somit betrifft die vorliegende Patentanmeldung auch ein Verfahren zur Herstellung des Verbundmaterials gemäß Anspruch 1 oder eines Formteils daraus umfassend die folgenden Schritte:

A) Bereitstellung eines körnigen Pulvers des Matrixwerkstoff;
B) Pressen eines Sinterrohling aus dem körnigen Pulver des Matrixwerkstoffs aus Schritt A);
C) Sintern des Sinterrohlings gemäß Schritt B) mit oder ohne Druckaufbringung durch Wärmebehandlung unterhalb der Schmelztemperatur des Matrixwerkstoffs zu einem Sinterling mit einer Porosität von 5% bis 30%;
D) Tauchen des Sinterlings gemäß Schritt C) unter Unterdruckbedingungen in geschmolzenes Silber bis die Poren mit Silber gefüllt sind;
E) Entnahme des Sinterlings gemäß Schritt D) aus dem flüssigen Silber und Abkühlen auf Raumtemperatur.

Vorzugsweise beträgt die Porosität gemäß Schritt C) zwischen YY% und 20%, weiter bevorzugt zwischen ZZ% und 15% und noch weiter bevorzugt zwischen ww% und 10%.

Vorzugsweise stelle man den Sinterrohling bereits in der Form des gewünschten Werkstücks oder Formteils her.

**Selektives Lasersintern:**

Ein neueres Verfahren ist die Herstellung der Sinterlinge durch das sogenannte Selektive Lasersintern (SLS), ein additives Fertigungsverfahren, um räumliche Strukturen durch Sintern mit einem Laserstrahl aus einem pulverförmigen Ausgangsstoff herzustellen.

Mithilfe eines Rakels wird eine Pulvermischung aus Matrixwerkstoff und Silber vollflächig in einer dünnen Schicht auf eine Bauplattform aufgebracht. Die Bauteilkontur wird schichtweise durch eine schnelle laterale Ablenkung des Laserstrahles in x- und y-Richtung, entsprechend der jeweiligen Schichtkontur des Bauteils, schrittweise in das Pulverbett gesintert oder eingeschmolzen. Die Strahlenergie des fokussierten Laserstrahls wird vom Pulver absorbiert und führt zu einem lokal begrenzten Sintern der Pulverpartikel. Die Bauplattform wird nach jedem Lasersinter-/-schmelzvorgang geringfügig abgesenkt und eine neue Pulverschicht aufgetragen. Das Werkstück wird somit Schicht für Schicht aufgebaut.

Durch Verwendung einer Pulvermischung aus einem Matrixwerkstoff wie z.B. Edelstahl mit dem gewünschten Silberpulveranteil kann ein abrasionsbeständiger Sinterling mit dem gewünschten antibakteriellen Silberanteil hergestellt werden. Dieses Verfahren bietet den Vorteil, den Silberanteil im erhaltenen Verbundmaterial sehr genau einzustellen, fast beliebige Formen zu erzeugen und eine raue Oberfläche auf dem Verbundmaterial zu generieren, welche beispielsweise für die Zellanhaftung und das Anwachsen von Knochen vorteilhaft ist.

Ferner betrifft die vorliegende Patentanmeldung ein Verfahren zur Herstellung eines Formteils aus dem Verbundmaterial gemäß Anspruch 1 umfassend die folgenden Schritte:

A) Bereitstellung eines Matrixwerkstoffs in Pulverform und Silber in Pulverform;
B) Herstellung einer Mischung, vorzugsweise einer homogenen Mischung, aus dem Pulver des Matrixwerkstoffs und einer definierten Menge an Silber in Pulverform;
C) Aufbringung einer dünnen Schicht der Mischung aus Schritt 2 auf eine Bauplattform;
D) Sintern der aufgebrachten dünnen Schicht gemäß Schritt C) mittels Laser;
E) Wiederholen der Schritte C) und D) so lange bis das Formteil aus dem Verbundmaterial erhalten wurde.

Das erfindungsgemäße Verbundmaterial mit Silberintegration soll vorzugsweise in der Kieferorthopädie für Zahnspangen-Brackets Anwendung finden. Aufgrund des hochfesten Matrixwerkstoffs widersteht das silberintegrierte Verbundmaterial der hohen abrasiven Belastung in der Mundhöhle. Überraschenderweise zeigt das Silber in den Poren eine so große antibakterielle "Fernwirkung", dass es nur zu einer sehr geringen bis gar keiner Anhaftung eines Biofilms kommt, auch wenn das Silber nicht die gesamte Oberfläche bedeckt wie bei einer Beschichtung. In einer klinischen Studie konnte dies anschaulich nachgewiesen werden wie unten weiter ausgeführt.

Die vorliegende Erfindung betrifft somit vorzugsweise die Zahnmedizin und soll eine Anlagerung von Bakterien an intraoral (in der Mundhöhle) befindliche Oberflächen verringern bzw. verhindern. Hierzu wurde ein Verbundmaterial mit integriertem Silber entwickelt. Der Matrixwerkstoff aus Metall oder Keramik sorgt für den Abrasionswiderstand und das integrierte Silber für die antibakterielle Wirkung. Die Ausbildung eines Biofilms sowie die einhergehenden negativen Folgen für die Zähne, den Zahnhalteapparat und weitere kollaterale Erkrankungen werden vermieden.

Nachgewiesen wurde der antibakterielle Effekt des erfindungsgemäßen Verbundmaterials im Bereich der Kieferorthopädie an Bracketmaterial für Zahnspangen. Die sonst entstehende dreidimensionale Biofilmstruktur aus Bakterien, eingebettet in einer Matrix aus extrazellulären Polysacchariden, wird direkt ab Einsetzen der festsitzenden Apparatur verhindert. Die Bakterien, die normalerweise die zugeführten Kohlenhydrate zu organischen Säuren verstoffwechseln und dadurch ein saures anaerobes Milieu bilden, sind nicht vorhanden. In der Folge werden keine Calcium- und Phosphationen aus der Kristallgitterstruktur des Zahnschmelzes herausgelöst. Durch die vorliegende Erfindung wird das initiale Stadium einer kariösen Läsion des Zahnes verhindert. Zudem sind klinisch keine weißlichen irreversiblen Entkalkungen des Zahnschmelzes mehr sichtbar.

Dass im Zuge des steigenden Gesundheitsbewusstseins und aus klinischer Notwendigkeit bestehende Bedürfnis, durch präventive Behandlungskonzepte eine schonende kieferorthopädische Behandlung durchzuführen, wird durch die zugrundeliegende Erfindung erfüllt.

Erfindungsgemäß widersteht das Bracketmaterial zudem den stark abrasiven Belastungen, denen kieferorthopädische Apparaturen durch Scherkräfte in der Mundhöhle ausgesetzt sind. Anders als bisherige oberflächlich aufgetragene Beschichtungen, die weich und stark abrasionsgefährdet sind, weist das entwickelte Verbundmaterial für z.B. Brackets von Zahnspangen erfindungsgemäß selbst antibakterielle Eigenschaften auf. Vor allem den starken Scherkräften in der Mundhöhle, bedingt durch die Zunge und Wange, halten die Brackets aus dem erfindungsgemäßen Verbundmaterial stand. Die antibakterielle Schutzwirkung ist, anders als bei oberflächlichen Beschichtungen, von Dauer. Die reguläre kieferorthopädische Behandlungszeit einer festsitzenden Therapie beträgt ca. 18 - 24 Monate. Für diesen gesamten Zeitraum ist eine antibakterielle Wirkung zur Verhinderung von "white spot lesions" durch das Verbundmaterial nachgewiesen.

Bisherige klinische Untersuchungen haben eindeutig gezeigt, dass die Bildung von Biofilmen auf dem erfindungsgemäßen Verbundmaterial mit integriertem Silber stark reduziert ist bzw. ganz verhindert wird. Das antibakteriell wirkende Silber wirkt, ohne dass eine geschlossene antibakterielle Oberflächenbeschichtung erforderlich ist.

Zudem haben die Untersuchungen des Abrasionsverhaltens gezeigt, dass die Biofilmbildung über den gesamten Therapiezeitraum signifikant reduziert ist. Das Verbundmaterial mit integriertem Silber hält den einwirkenden Scherkräften im Mundraum stand.

Auch wenn es bei Langzeitanwendungen in der Mundhöhle zu Abrieb des Matrixwerkstoffs kommt, ist durch die Silberintegration im Basismaterial stets genügend Silber an der Porenoberfläche vorhanden, um die antibakterielle Wirkung sicherzustellen. Hierin besteht auch der deutliche Vorteil gegenüber den herkömmlichen antibakteriellen Beschichtungen, welche der abrasiven Belastung nicht lange standhalten, so dass eine anfänglich deutliche antibakterielle Wirkung recht zügig verlorengeht. Die Integration und homogene Verteilung des Silbers in dem Matrixwerkstoff und damit in dem Verbundmaterial garantiert auch bei stärkerem Abrieb eine gleichbleibend gute und vor allem dauerhafte antibakterielle Wirkung.

Damit existiert erstmalig ein Material, vorzugsweise für die Zahnmedizin, mit antibakterieller Langzeitwirkung und hoher Abrasionsbeständigkeit. Insbesondere in der Kieferorthopädie und dort vorzugsweise für Multibracketapparaturen werden Dekalzifikationen des Zahnschmelzes und in der Folge Karies auch bei Langzeitanwendung vermieden.

Dementsprechend betrifft die vorliegende Erfindung medizinische Implantate, welche das erfindungsgemäße Verbundmaterial enthalten oder aus dem Verbundmaterial bestehen.

Die hierin beschriebenen medizinischen Implantate, welche aus dem Verbundmaterial bestehen oder die hierin beschriebenen medizinischen Implantate, welche das Verbundmaterial oder Komponenten aus dem Verbundmaterial enthalten, sind insbesondere geeignet, Scherkräften und Abrasionskräften ausgesetzt zu werden.

Bei diesen medizinischen Implantaten kann es sich um medizinische Kurzzeitimplantate oder medizinische Langzeitimplantate und vorzugsweise um dentale Langzeitimplantate handeln.

In der Zahnmedizin sind auch andere Anwendungen für dieses neue Verbundmaterial vorgesehen z.B. Verankerungsapparaturen, wie Transpalatinalbögen und Lingualbögen, die ebenfalls aus antibakteriellen Werkstoffen bestehen sollten. Auch eingesetzte Mini-Schrauben, die zur Verankerung mit dem Gewinde in dem Kieferknochen fixiert werden, während der Schraubenkopf in der Mundhöhle exponiert bleibt, sind ein wichtiges Anwendungsgebiet der vorliegenden Erfindung. Zahninlays aus Keramik und metallische oder keramische Zahnkronen sind ein weiteres Einsatzgebiet des Verbundmaterials.

Somit sind Beispiele für medizinische Implantate insbesondere Dentalimplantate aller Art wie Brackets, kieferorthopädische Bögen, Ligaturen und Bänder, dentale Verankerungsapparaturen wie Transpalatinalbögen und Lingualbögen, Temporary anchorage devices (z.B. Minipins, Gaumenimplantate, Bollard Pins), Mini-Schrauben, Zahninlays sowie metallische oder keramische konfektionierte Zahnkronen.

Den genannten Apparaturen, Mechaniken und Implantaten der Zahnmedizin ist gemein, dass sie für eine lange Zeitspanne (Monate bis Jahre) im intraoralen Bereich fixiert werden und somit den oralen Bedingungen ausgesetzt sind.

Dentalimplantate wie beispielsweise Brackets, Transpalatinalbögen, Lingualbögen, Mini-Schrauben, Zahninlays sowie metallische oder keramische Zahnkronen können vollständig aus dem Verbundmaterial gefertigt werden oder nur bestimmte Bereiche enthalten, die aus dem Verbundmaterial gefertigt worden sind. Bei Zahnspangen als Dentalimplantat sind in der Regel nur die Brackets aus dem Verbundmaterial hergestellt.

Auch wenn das hierin offenbarte Verbundmaterial vorzugsweise für Dentalimplantate Verwendung findet, können auch andere Medizinprodukte und vor allem andere Implantate, welche auch starken Kräften oder Drücken ausgesetzt sind, daraus gefertigt werden. So kann das Verbundmaterial mit integriertem Silber als Implantatwerkstoff in der Orthopädie angewandt werden, um Entzündungen bei z.B. Knieimplantaten zu verhindern. Beispiele für weitere Medizinprodukte sind orthopädisches Implantate wie beispielsweise Gelenkimplantate vor allem Knieimplantate, Zwischenwirbelimplantate, Knochenkeile oder Knochenschrauben.

Der große Vorteil des erfindungsgemäßen Verbundmaterials ist, dass dieses Verbundmaterial an sich antibakteriell ist und sich somit eine antibakterielle Beschichtung erübrigt und damit auch ein weiterer Arbeitsschritt der Aufbringung einer antibakteriellen Beschichtung auf das medizinische Implantat oder Teilen davon.

Die vorliegende Erfindung ist das Ergebnis intensiver Biofilmforschung. Alle bisherigen Werkstoffe bzw. Werkstoff- oder Oberflächenmodifikationen zeigten in zahlreichen klinischen Studien keine zufriedenstellende antibakterielle Langzeitwirkung.

Dagegen weist das Verbundmaterial mit Silberintegration eine starke antibakterielle Langzeitwirkung auf und beugt somit der Entstehung eines Biofilms vor oder verhindert ihn vollständig. Überraschenderweise ist keine geschlossene Silberoberfläche erforderlich wie bei einer Beschichtung, um einen Biofilm umfassend zu verringern oder zu verhindern. Selbst Verbundmaterialien mit einem Silbergehalt von weniger als 20%, deren Zwischenräume mit Silber gefüllt sind, zeigen eine hervorragende antibakterielle Langzeitwirkung.

Die vorliegende Erfindung hat einen neuartigen Ansatz verfolgt, um die Biofilmbildung z.B. auf kieferorthopädischen festsitzenden Apparaturen effektiv zu verringern und langfristig einen antibakteriellen Effekt zu erzeugen.

Für das erfindungsgemäß entwickelte Verbundmaterial mit integriertem Silber konnte nachgewiesen werden, dass selbst bei Silbergehalten von unter 20 %, vorzugsweise unter 15% stark antibakterielle Eigenschaften vorliegen, bei Silbergehalten von unter 10%, aber einem Mindestsilbergehalt von 5% noch hinreichende antibakterielle Eigenschaften vorliegen und diese antibakterielle Eigenschaft auch unter abrasiven Bedingungen weiterbesteht. Zudem ist das Verbundmaterial biokompatibel.

Bei Anwendung für kieferorthopädisches Bracketmaterial unterscheidet sich das erfindungsgemäße Verbundmaterial maßgeblich von den bisherigen, auf dem Markt befindlichen Bracketmaterialien. Durch seine inhärente antibakterielle Wirkung sind zusätzliche antibakterielle Beschichtungen der Brackets nicht notwendig. Damit verbindet die vorliegende Erfindung erstmals antibakterielle Eigenschaften mit einem langanhaltenden antibakteriellen Schutz zur Verhinderung eines intraoralen Biofilms.

Somit weist die Erfindung folgende Vorteile auf:

- antibakterielle Wirkung,
- hohe Abrasionsbeständigkeit,
- langanhaltende antibakterielle Wirkung über die gesamte Anwendungsdauer,
- gleichmäßige Silberintegration im Verbundmaterial, auch im Kantenbereich,
- Gesamtmodifikation des Verbundmaterials anstelle einer oberflächlich aufgetragenen Beschichtung,
- keine Erhöhung der Bracketdicke,
- Biokompatibilität,
- geringer Verbrauch an antibakteriellem Silber,
- unkomplizierter Herstellungsprozess, daher für Produktion von großen Stückzahlen geeignet.

## Figurenbeschreibung

**Figur 1:** zeigt eine Rasterelektronenmikroskopaufnahme eines Querschliffes eines porösen Matrixwerkstoffs (hier Wolfram) mit infiltriertem homogen verteiltem Silber (schwarz).

**Figur 2:** zeigt eine Zahnputzmaschine zur Bestimmung der Abrasionsbeständigkeit von Brackets aus herkömmlichem Edelstahl und aus Wolfram mit infiltriertem homogen verteiltem Silber.

**Figur 3:** zeigt die Wandstärke vor und nach Abrasion bei einem Bracketmaterial aus herkömmlichem Edelstahl ("Kontrolle") und einem Verbundmetall aus Wolfram als Matrixwerkstoff mit niedriger (Low Ag = 7-9 Gew.-% Ag im Matrixwerkstoff) bzw. hoher (High Ag = 9-14 Gew.-% Ag im Matrixwerkstoff) Silberintegration. Bei dem Verbundmaterial aus Wolfram mit Silberintegration wurde ein geringer Materialabtrag festgestellt, ebenso wie zu Vergleichszwecken bei den Proben aus konventionellen Edelstahlbrackets ("Kontroll" Gruppe).

**Figur 4:** zeigt ein signifikant reduziertes Biofilmvolumen auf intraoral getragenem Bracketmaterial aus silberintegriertem Wolfram-Verbundmetall (Kontrolle = Edelstahl, Low Ag = 7-9% Ag, High Ag = 9-14% Ag im Verbundmetall.

**Figur 5:** Biofilm auf im Mund getragenen Bracketproben gemäß Beispiel 4

## Beispiele

**Beispiel 1:** Herstellung des Verbundmaterials gemäß Figur 1

Bei der Herstellung des Wolframverbundmaterials wurden verschieden feinkörnige Fraktionen von Wolframpulver (verschiedene Körnungen) zusammengegeben und über einen längeren Zeitraum gemischt, um eine gleichmäßige Verteilungen der Körnungen im Pulver zu erhalten.

Zur Formgebung wurde die Pulvermischung in Werkzeugen unter hohem Druck zu sogenannten "Grünlingen" verpresst. Die Grünlinge haben dabei schon die Form des Endproduktes. Im Fall der hergestellten Brackets wurden dünne Ronden mit einem Durchmesser von 20 mm und einer Wandstärke von 2 mm hergestellt.

Anschließend wurden die Ronden (Grünlinge) gesintert, d.h. unter hohen Temperaturen, die unterhalb der Schmelztemperaturen hier von Wolfram liegen, "verbacken". Der Sintervorgang verläuft unter Schutzgasatmosphäre oder im Vakuum ab.

Die fertigen Sinterlinge wurden anschließend in flüssiges Silber getaucht. Dies geschieht im Vakuum, damit sich alle Poren vollständig mit Silber füllen.

Die silberhaltigen Ronden wurden auf eine Wandstärke von 0,6 mm beidseitig abgeschliffen und mittels Laser auf die Außenabmessungen der Brackets zugeschnitten.

**Beispiel 2:** Rasterelektronenmikroskopaufnahme gemäß Figur 1

Die Brackets wurden auf einem Probenhalter befestigt und in die Probenkammer des Rasterelektronenmikroskops der Firma TESCAN Typ Vega3 - ohne weitere Probenvorbereitung - gegeben. Die Aufnahme des Sintergefüges ist im sogenannten BSE-Verfahren (backscattered electrons) entstanden, welches einen guten Materialkontrast ermöglicht. So stellen in Figur 1 die hellen Bereiche die Wolframkörner dar, die von dunklem (schwarzem) Silber umgeben sind.

**Beispiel 3:** Abrasionsuntersuchungen an einem silberintegriertem Verbundmaterial

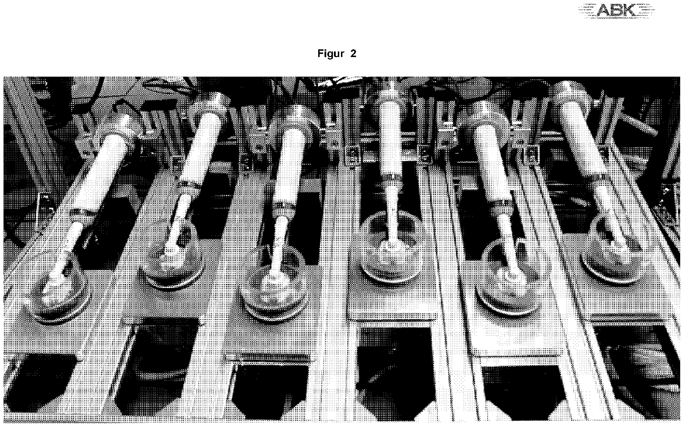

Um die Abrasionsbeständigkeit zu testen, wurden an Proben aus konventionellen Edelstahlbrackets und zum Vergleich aus einem silberintegrierten Verbundmetall aus silberinfiltriertem Wolfram Dauerversuche mit einer Zahnputzmaschine durchgeführt (siehe Fig. 2). Mit rotierenden Zahnbürsten, einer oszillierenden linearen Relativbewegung zwischen Zahnbürste und Metalloberfläche sowie unter Zugabe von abrasiver Zahnpasta wurde der Zahnputzvorgang für einen Zeitraum von 2 Jahren simuliert.

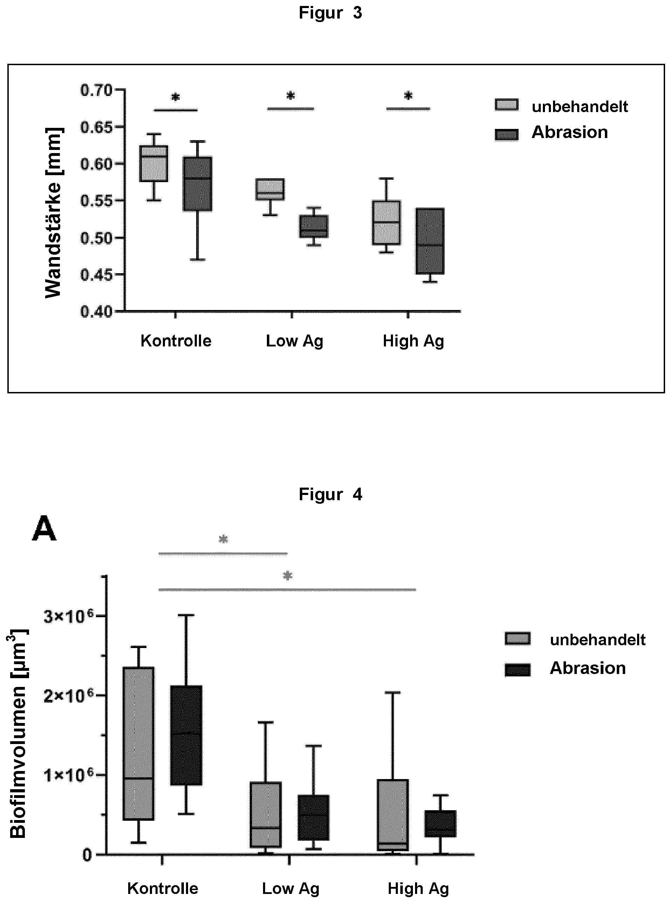

Bei dem Verbundmaterial aus Wolfram mit Silberintegration wurde ein geringer Materialabtrag festgestellt, ebenso wie zu Vergleichszwecken bei den Proben aus konventionellen Edelstahlbrackets ("Kontroll" Gruppe), siehe Fig. 3.

Der Materialabtrag von weniger als 50 $\mu$m in der Bracketwandstärke ist für eine Tragedauer von 2 Jahren akzeptabel. Die Brackets aus Verbundmaterial sind damit ausreichend abrasionsbeständig. Entscheidend ist, dass bei jedem Abtrag in den Poren befindliches Silber freigelegt wird, d.h. an der Bracketoberfläche vorhanden ist und für einen dauerhaften antibakteriellen Effekt sorgt.

**Beispiel 4:** Klinische Studie zum Nachweis der antibakteriellen Wirkung bei Brackets

In einer klinischen Studie wurden intraoral Trägerschienen getragen, die mit Brackets aus konventionellem Edelstahl und im Vergleich aus Verbundmetall (hier Wolfram) mit Silberintegration bestückt waren.

Das für die Studie hergestellte neuartige Verbundmaterial aus Wolfram mit einer Silberintegration lag anfangs in Ronden mit einem Durchmesser von 19.3 mm und einer Dicke von 2.0 mm vor. Mittels einer Bandschleifanlage wurden die Ronden auf eine durchschnittliche Wandstärke von 0.64 mm reduziert. Anschließend wurden die Proben mittels Laser auf eine Kantenlänge von 5.0 x 5.0 mm zugeschnitten.

Als Kontrollgruppe wurden Probekörper aus konventionell erhältlichem EdelstahlBracketmaterial der Werkstoffnummer 1.4456 herangezogen und auf die gleiche Probengröße zugeschnitten. Die exakte Materialzusammensetzung der Verbundmetall-Probekörper und der Kontroll-Probekörper wurde mittels der energiedispersiven Röntgenspektroskopie (EDX) ermittelt. Die Oberflächenrauheit der unterschiedlichen Probekörper wurde mittels konfokaler Laser-Scanning-Mikroskopie (CLSM) gemessen. Somit wurde sichergestellt, dass alle Proben eine mittlere Rauheit (Ra) unter dem Grenzwert von Ra = 0,2 $\mu$m aufwiesen, ab dem ein Einfluss der Bakterienanhaftung durch zu raue Oberflächenstruktur eines Materials nachzuweisen ist. Die Unterschiede in der Biofilmbildung konnten somit ausschließlich auf das Probenmaterial selbst zurückgeführt werden.

Für die vorliegende Studie wurde eine Zahnputzmaschine designt und gebaut, um die abrasive Belastung der Brackets durch das tägliche Zähneputzen nachzubilden. Dabei wurde das zweimal tägliche Putzen für jeweils zwei Minuten bei einer durchschnittlichen Tragezeit der Brackets von 24 Monaten angenommen. Dieser Zeitraum konnte mittels der konstruierten Zahnputzmaschine durch eine kontinuierliche zweistündige Behandlung der Proben reproduziert werden.

Um die gewonnenen Daten aus der vorliegenden Studie ausschließlich auf den neu entwickelten Bracketwerkstoff zurückführen zu können, wurden durch das Studiendesign möglichst gleiche Ausgangsbedingungen geschaffen. In die klinische Studie wurden zwölf parodontal gesunde Probanden (sechs Frauen und sechs Männer) im Alter zwischen 21 und 30 Jahren eingeschlossen. Alle zwölf Probanden konnten unter Einhaltung des erstellten Versuchsprotokolls in die Studie einbezogen werden. Mittels eines parodontalen Screenings der zwölf Probanden wurde überprüft, dass die Studie unter parodontal gesunden oralen Bedingungen durchgeführt wird. Hierzu wurden verschiedene parodontale Indizes, u.a. der approximale Plaqueindex als Indikator für die vorherrschende Mundhygiene, erhoben. Weitere Risikokriterien, die eine Zusammensetzung der mikrobiellen Flora verändern, wurden ausgeschlossen. Hierzu zählen Schwangerschaft, Rauchen, Allgemeinerkrankungen, herausnehmbarer Zahnersatz und eine mögliche Antibiotikaeinnahme in den letzten sechs Wochen vor Studienbeginn.

Für die zwölf Probanden wurde eine Miniplastschiene aus Kunststoff im Tiefziehverfahren hergestellt. Um eine fälschliche Reduktion des Biofilms durch bestehende Scherkräfte in der Mundhöhle sowie durch Reiben von Wange und Zunge zu verhindern, wurden an der Miniplastschiene Pelotten aus Kunststoff im Seitenzahnbereich angebracht. Diese Pelotten wurden mit einem kleinen Abstand zur Miniplastschiene im Sprüh- und Streuverfahren an einem Haltedraht befestigt, sodass die Speichelzirkulation zwischen der Schiene und der abhaltenden Pelotten einwandfrei möglich war. Die Schienen wurden anschließend mit den zu untersuchenden Probekörpern bestückt. Um die Haftung des fließfähigen Komposits als Verbundwerkstoff zwischen den Probekörpern und der Miniplastschiene zu erhöhen, wurden die Klebestellen auf den Schienen mittels Sandpapier angeraut. Auf beiden Schienenseiten wurde in randomisierter Reihenfolge eine Kontrolle aus konventionellem Edelstahl, eine Probe aus dem Verbundmetall (hier Wolfram) mit einer Silberintegration von 20% und eine Probe aus dem Verbundmetall (hier Wolfram) mit einer Silberintegration von 15% fixiert. Die Reihenfolge der drei Probekörper pro Schienenseite erfolgte randomisiert, da die Biofilmbelegung innerhalb der Mundhöhle je nach Position variieren kann. Somit wurde ein möglicher Effekt der Probenposition ausgeschlossen.

Zur Bestimmung der Tragezeit, die eine reproduzierbare Auswertung des intraoral gebildeten Biofilms ermöglicht, wurde in mehreren Vorversuchen an Probanden eine Tragedauer von 48 Stunden festgelegt. Während dieser Zeit musste die Mundhygiene und der Alkoholkonsum ausgesetzt werden, um keine Verfälschung des gebildeten Biofilms durch Reinigung der Probekörper zu provozieren. Zum Essen wurden die Schienen für maximal 40 Minuten ausgegliedert und in einem feuchten Milieu gelagert.

Nach Beendigung der Tragezeit wurden die Schienen den Probanden entnommen und der gebildete Biofilm mit einer Lebend/Tot-Fluoreszenzfärbung markiert sowie mit Glutardialdehyd fixiert. Die dreidimensionale quantitative Analyse des fluoreszenzgefärbten Biofilms erfolgte mittels konfokaler Laser-Scanning-Mikroskopie (CLSM). Die statistische Auswertung der erhobenen Daten erfolgte mit Hilfe der Software GraphPad Prism bei einem Signifikanzlevel von $p \leq 0.05$.

Es konnte nachgewiesen werden, dass die Bracketproben aus dem silberintegrierten Wolfram-Verbundmetall im Vergleich zu dem konventionellen Edelstahlbracketmaterial ein signifikant reduziertes Biofilmvolumen aufwiesen:

a) Für unbehandelte Bracketproben beträgt das Biofilmvolumen für die Standardbrackets aus Edelstahl im Mittel 1.000.000 $\mu m^3$ und bei den Brackets aus silberintegrierten Verbundmetall im Mittel nur 280.000 $\mu m^3$ für LowAg bzw. 120.000 $\mu m^3$ für HighAg (siehe Fig. 3).

b) Für Brackets, die zuvor in der Zahnputzmaschine eine Abrasion erfahren haben, beträgt das Biofilmvolumen für die Standardbrackets aus Edelstahl im Mittel 1.600.000 $\mu m^3$ und bei den Brackets aus silberintegrierten Verbund-metall im Mittel nur $\pm$ 420.000 $\mu m^3$ für LowAg bzw. 230.000 $\mu m^3$ für HighAg (siehe Fig. 4).

Fig. 4 zeigt, dass auch bei Bracketmaterialproben, die zuvor einer Abrasion in der Zahnputzmaschine (s. Fig. 2) ausgesetzt waren, eine deutliche Reduktion des Biofilmvolumens vorliegt, d.h. die Wirkung des im Matrixwerkstoff integrierten Silbers bleibt trotz Abrasion der Randschicht dauerhaft erhalten.

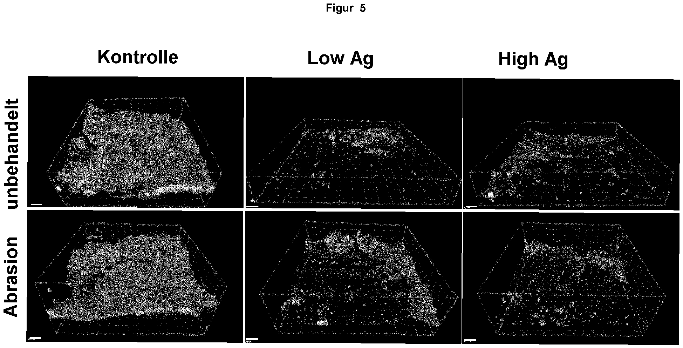

Die antibakterielle Wirkung des Silbers im Verbundmaterial zeigt sich deutlich, wenn der Biofilm, bestehend aus einer Vielzahl von Bakterien, nach der intraoralen Tragezeit fluoreszenzgefärbt und anschließend unter einem konfokalen Laser-Scanning-Mikroskop ausgewertet wird. Fig. 5 zeigt in hell (**Grün**) lebende und in dunkel (**Rot**) tote Bakterien.

Bei den Standardbrackets aus Edelstahl bilden sich beim Tragen in der Mundhöhle geschlossene Biofilme aus, sowohl für unbehandelte Brackets als auch für Brackets, die zuvor einer Abrasion in der Zahnputzmaschine unterzogen wurden (siehe Fig. 5 "Kontroll" Gruppe).

Hingegen ist beim Verbundmaterial mit wenig Silber (7-9 Gew.-% Ag) und mit viel Silber (9-14 Gew.-% Ag) nur eine geringe Biofilmausbildung zu sehen (siehe Fig. 5). Auch nach Abrasion in der Zahnputzmaschine liegt nur eine geringe Biofilmausbildung vor, d.h. dass im Material integrierte Silber ist wirksam.

Diese geringe Biofilmausbildung ist umso bedeutender, da bei der klinischen Studie die Zähne bzw. Bracketproben für 48 Stunden nicht geputzt wurden und trotzdem kaum Biofilm vorhanden ist.

**Patentansprüche**

1. Verbundmaterial aus einem Matrixwerkstoff und Silber, wobei das Silber homogen in dem Matrixwerkstoff verteilt ist und der Gehalt an Silber im Verbundmaterial zwischen 5 Gew.-% und 40 Gew.-% liegt.

2. Verbundmaterial gemäß Anspruch 1, wobei der Matrixwerkstoff ausgewählt ist aus im Dentalbereich eingesetzte Metalle und Metalllegierungen, vorzugsweise austenitische Metalle und austenitische Metalllegierungen.

3. Verbundmaterial gemäß Anspruch 1 oder 2, wobei der Matrixwerkstoff ausgewählt ist aus der Gruppe bestehend aus oder umfassend medizinischem Edelstahl, CoCr-Legierungen, Eisen-Chrom-Mangan-Legierungen, Cobalt-Nickel-Chrom-Legierungen, Eisen-Chrom-Nickel-Legierungen, Eisen-Cobalt-Nickel-Legierungen, Titanlegierungen, Tantal, Wolfram, Keramiken, Aluminiumoxid und Zirkoniumdioxid.

4. Verbundmaterial gemäß einem der Ansprüche 1 - 3 zur Reduzierung oder Verhinderung der Ausbildung und Anhaftung eines Biofilms an der Oberfläche des Verbundmaterials.

5. Verbundmaterial gemäß einem der Ansprüche 1 - 4, wobei der Gehalt an Silber im Verbundmaterial zwischen 9 Gew.-% und 17 Gew.-% liegt.

6. Verfahren zur Herstellung eines Formteils aus dem Verbundmaterial gemäß einem der Ansprüche 1 - 5 umfassend die folgenden Schritte:

   A) Bereitstellung eines Matrixwerkstoffs in Pulverform und Silber in Pulverform;
   B) Herstellung einer Mischung, vorzugsweise einer homogenen Mischung, aus dem Pulver des Matrixwerkstoffs und einer definierten Menge an Silber in Pulverform;
   C) Aufbringung einer dünnen Schicht der Mischung aus Schritt 2 auf eine Bauplattform;
   D) Sintern der aufgebrachten dünnen Schicht gemäß Schritt C) mittels Laser;
   E) Wiederholen der Schritte C) und D) so lange bis das Formteil aus dem Verbundmaterial erhalten wurde.

7. Verfahren zur Herstellung des Verbundmaterials gemäß einem der Ansprüche 1 - 5 umfassend die folgenden Schritte:

   A) Bereitstellung eines körnigen Pulvers des Matrixwerkstoff;
   B) Pressen eines Sinterrohling aus dem körnigen Pulver des Matrixwerkstoffs aus Schritt A);
   C) Sintern des Sinterrohlings gemäß Schritt B) mit oder ohne Druckaufbringung durch Wärmebehandlung unterhalb der Schmelztemperatur des Matrixwerkstoffs zu einem Sinterling mit einer Porosität von 5% bis 30%;
   D) Tauchen des Sinterlings gemäß Schritt C) unter Unterdruckbedingungen in geschmolzenes Silber bis die Poren mit Silber gefüllt sind;
   E) Entnahme des Sinterlings gemäß Schritt D) aus dem flüssigen Silber und Abkühlen auf Raumtemperatur.

8. Medizinisches Implantat enthaltend oder bestehend aus dem Verbundmaterial gemäß einem der Ansprüche 1 - 7.

9. Medizinisches Implantat gemäß Anspruch 8, wobei das Verbundmaterial im medizinischen Implantat Scherkräften und Abrasionskräften ausgesetzt ist.

10. Medizinisches Implantat gemäß Anspruch 8 oder 9, wobei es sich bei dem medizinischen Implantat um ein medizinisches Kurzzeitimplantat oder ein medizinisches Langzeitimplantat handelt.

11. Medizinisches Implantat gemäß einem der Ansprüche 8 - 10, wobei es sich bei dem medizinischen Implantat um ein Dentalimplantat, Bracket, kieferorthopädischen Bogen, Ligatur, Band, dentale Verankerungsapparatur, Transpalatinalbogen, Lingualbogen, Temporary anchorage device, Minipin, Gaumenimplantat, Bollard Pin, Mini-Schraube, Zahninlay, metallische oder keramische konfektionierte Zahnkrone handelt.

12. Medizinisches Implantat gemäß einem der Ansprüche 8 - 10, wobei es sich bei dem medizinischen Implantat um ein orthopädisches Implantat, Gelenkimplantat, Zwischenwirbelimplantat, Knochenkeil oder Knochenschraube handelt.

13. Medizinisches Implantat gemäß einem der Ansprüche 8 - 12, wobei das Verbundmaterial im medizinischen Implantat keine antibakterielle Beschichtung aufweist.

**Figur 1**

| SEM HV: 30.0 kV | WD: 15.00 mm | | VEGA3 TESCAN |
| View field: 254 µm | Det: BSE | 50 µm | |
| SEM MAG: 2.00 kx | Date(m/d/y): 12/06/19 | | MeKo - laser material processing |

ABK

Figur 2

Figur 3

Figur 4

Figur 5

Europäisches
Patentamt

European
Patent Office

Office européen
des brevets

**EUROPÄISCHER RECHERCHENBERICHT**

Nummer der Anmeldung

EP 21 16 4744

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 111 197 130 A (NANTONG JINYUAN INTELLIGENCE MANUFACTURING TECH CO LTD) 26. Mai 2020 (2020-05-26) * Ansprüche 1, 3 * * Absätze [0003], [0042] * ----- | 1-13 | INV. B22F3/06 A61C7/12 A61F2/28 A61F2/30 A61K6/844 |
| X | CN 102 648 876 B (BEIJING SMART TECHNOLOGY CO LTD) 26. August 2015 (2015-08-26) * Ansprüche 1, 3 * ----- | 1-5, 8-11,13 | B22F3/12 B22F3/26 B22F10/28 C22C1/04 |
| X | JIANG FEILONG ET AL: "A strong, wear- and corrosion-resistant, and antibacterial Co-30 at.% Cr-5 at.% Ag ternary alloy for medical implants", MATERIALS & DESIGN, ELSEVIER, AMSTERDAM, NL, Bd. 184, 6. September 2019 (2019-09-06), XP086033310, ISSN: 0264-1275, DOI: 10.1016/J.MATDES.2019.108190 [gefunden am 2019-09-06] * Zusammenfassung * * 1. Einführung, erster Absatz * ----- | 1-4, 8-10,12, 13 | C22C1/05 C22C29/12 |

RECHERCHIERTE SACHGEBIETE (IPC)

B22F
A61F
C22C
A61C
A61K

Der vorliegende Recherchenbericht wurde für alle Patentansprüche erstellt

| Recherchenort | Abschlußdatum der Recherche | Prüfer |
|---|---|---|
| Den Haag | 2. August 2021 | Gomes Pinto F., R |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 16 4744

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-08-2021

| Im Recherchenbericht angeführtes Patentdokument | Datum der Veröffentlichung | Mitglied(er) der Patentfamilie | Datum der Veröffentlichung |
|---|---|---|---|
| CN 111197130 A | 26-05-2020 | KEINE | |
| CN 102648876 B | 26-08-2015 | KEINE | |

Für nähere Einzelheiten zu diesem Anhang : siehe Amtsblatt des Europäischen Patentamts, Nr.12/82